# EUROPEAN PATENT APPLICATION

(11) **EP 3 705 722 A1**
(43) Date of publication of application: **09.09.2020**
(21) Application number: 17931020.6
(22) Date of filing: 11.12.2017
(51) Int. Cl.: F04B 49/06, F04B 51/00

(54) **HIGH-PRECISION PERISTALTIC PUMP FLOW RATE CONTROL SYSTEM**

(30) Priority: 31.10.2017 CN 201721434944 U
(71) Applicant: Sichuan Nigale Biotechnology Co., Ltd, Chengdu, Sichuan 641403 (CN)
(72) Inventor: PENG, Chun, Chengdu Sichuan 641403 (CN); WANG, Chaofu, Chengdu Sichuan 641403 (CN)
(74) Representative: Ipside
(86) International application number: PCT/CN2017/115385
(87) International publication number: WO 2019/085155

(57) **Abstract**

The utility model discloses a high-precision peristaltic pump flow rate control system. The high-precision peristaltic pump flow rate control system comprises a motor, a peristaltic pump, a rotational speed feedback assembly, a flow rate feedback assembly and a controller. The motor is correspondingly connected with the peristaltic pump, the peristaltic pump is correspondingly connected with the rotational speed feedback assembly and the flow rate feedback assembly, the rotational speed feedback assembly and the flow rate feedback assembly are correspondingly connected with the controller, and the controller is correspondingly connected with the motor. The flow rate feedback assembly comprises a flow rate measurement means and a determining module. The flow rate of the anticoagulant pump and the flow rate of the blood pump are made steady near the set point by means of the flow rate feedback assembly. In this way, the precision control of the anticoagulant dosage for plasmapheresis is realized to improve the quality of plasmapheresis and reduce the impact on the health of the plasma donor.

## Description

### Technical Field

The utility model relates to the field of plasma collection, in particular to a peristaltic pump flow rate control system.

### Background of the Invention

To properly preserve the human blood in vitro for a long time, the currently used blood preserving fluid acts as the anticoagulant by binding citrate with calcium ions in the blood into soluble and non-ionizable calcium citrate. Therefore, free calcium is converted into bound calcium and physiologically loses the coagulation function, and the blood is left incoagulable in vitro.

During plasmapheresis process, the blood is mixed with the anticoagulant (at a ratio of **16:1**, fine-tuned depending on individual **HCT**), the plasma is separated out from resulting mixture by means of centrifugal separation treatment, and erythrocytes, buffy coat and the like are transfused back to the plasma donor with the anticoagulant. If the blood with excess blood preserving fluid is transfused to the patient, the excess citrate may continue to bind with the calcium ions in the patient's blood to decrease the calcium concentration and cause hypocalcemia. If the blood with excess blood preserving fluid is transfused to children or a patient receives the transfusion from a plurality of bags containing the blood, in particular to a patient at a low calcium level of blood, serum calcium would reduce and clinical manifestation of calcium deficiency is induced. In addition, if the blood with excess anticoagulant is transfused to a patient, the normal coagulation will be prolonged in such a way that it affects the rehabilitation of the patient.

For plasmapheresis equipment at home and abroad, the anticoagulant and blood are mixed by means of motor-driven peristaltic pump technology. The rotational speed of the peristaltic pump motor is subject to the speed feedback control through an encoder of a servo motor or a stepping motor combined with Hall sensor. By default, the blood is drawn at a consistent flow rate (e.g., **1mL/**revolution) by the peristaltic pump, and the mixing ratio of the anticoagulant to the blood is controlled by setting the rotational speed ratio of the peristaltic pump (e.g., when the rotational speed ratio of the blood pump to the anticoagulant pump is set to **16:1**, the mixing ratio of the blood to the anticoagulant reaches **16:1**).

The motor control technology of prior art is relatively well-developed, and a plurality of technical means are available for realizing precision control of the motor rotational speed. The flow volume per revolution of the motor is constant by default in the application scenario of plasmapheresis. However, in the actual application process, the flow volume per revolution is frequently affected by a series of factors, including wear of peristaltic pump roller, difference among individual piping consumables and line pressure. In other words, with about 20% deviation in the flow volume per revolution of the peristaltic pump, the actual dosage of the anticoagulant has a deviation of about **20%.**

### Summary of the Invention

The utility model discloses a high-precision peristaltic pump flow rate control system to solve the technical problem.

The technical solution of the utility model is as follow:
As a high-precision peristaltic pump flow rate control system, an embodiment of the utility model comprises a motor, a peristaltic pump, a rotational speed feedback assembly, a flow rate feedback assembly and a controller. The motor is correspondingly connected with the peristaltic pump. The peristaltic pump is correspondingly connected with the rotational speed feedback assembly and the flow rate feedback assembly, the rotational speed feedback assembly and the flow rate feedback assembly are correspondingly connected with the controller, the controller is correspondingly connected with the motor, and the flow rate feedback assembly comprises a flow rate measurement means and a determining module.

Further, the flow rate measurement means is a flow transducer.

Other aspects and advantages of the utility model will become apparent from the following detailed description in combination with the drawings which illustrate, by means of embodiment, the principles of the utility model.

### Brief description of the Drawings

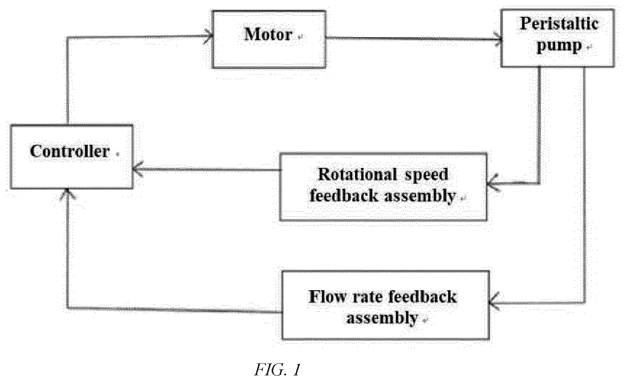

Fig. 1 shows a structural block diagram of a peristaltic pump flow rate control system provided by the embodiment of the utility model.

### Detailed Description of Embodiments

The utility model will be described in detail in combination with drawings.

The utility model will be further described in detail in combination with drawings and embodiments for clear understanding of the purpose, technical solution and advantages of the utility model. It should be understood that various embodiments described herein are only used to explain the utility model rather than defining the utility model.

The utility model discloses a high-precision peristaltic pump flow rate control system with a flow rate feedback mechanism.

The utility model discloses a high-precision peristaltic pump flow rate control system, comprising a motor, a peristaltic pump, a rotational speed feedback assembly, a flow rate feedback assembly and a controller. The motor is correspondingly connected with the peristaltic pump, the peristaltic pump is correspondingly connected with the rotational speed feedback assembly and the flow rate feedback assembly, the rotational speed feedback assembly and the flow rate feedback assembly are correspondingly connected with the controller, and the controller is correspondingly connected with the motor. The flow rate feedback assembly comprises a flow rate measurement means and a determining module.

The peristaltic pump of the utility model comprises an anticoagulant pump and a blood pump. The anticoagulant pump is arranged in an anticoagulant tube to drive an anticoagulant into the anticoagulant tube; and the blood pump is arranged in a blood tube to drive blood into the blood tube. The anticoagulant pump and the blood pump are conventional devices for plasma collection.

The flow rate measurement means of the utility model detects a flow rate of the anticoagulant pump and the blood pump at a set rotational speed and uploads the detected flow rate to the determining module. If the determining module decides that the flow rate of the anticoagulant pump or the blood pump is less than a set point at the rotational speed, it indicates an insufficient flow rate due to a low roller extrusion force, an overvalued speed feedback and other factors. The determining module sends the decision result to a control device, and the control device increases the flow rate to the set point by controlling corrective actions for increasing the rotational speed of the anticoagulant pump or the blood pump. Similarly, if the determining module decides that the flow rate of the anticoagulant pump or the blood pump is higher than the set point at the rotational speed, it indicates an excess flow rate due to a high roller extrusion force, an undervalued speed feedback and other factors. The determining module sends the decision result to the control device, and the control device decreases the flow rate to the set point by controlling the corrective actions for reducing the rotational speed of the anticoagulant pump or the blood pump.

The flow rate measurement means of the utility model may be a flow transducer. The flow transducer is respectively arranged in an anticoagulant pump tube and a blood pump tube to monitor the flow rate of the anticoagulant pump and the flow rate of the blood pump respectively. Accuracy of the flow transducer for flow monitoring has a direct influence on control of the anticoagulant dosage. A self-calibration module is arranged in the flow transducer to ensure effectiveness of the flow monitoring. Preferably, the flow transducer is a small-diameter high-resolution ultrasonic flow transducer with an inner diameter of 3 to 5 mm and an outer diameter of 6 to 7 mm. Therefore, a flow transducer with an inner diameter of 4 mm, an outer diameter of 6.4 mm and at a resolution of 1.25 ml/min is adopted in an embodiment.

The flow rate measurement means of the utility model may comprise a counting device and a volume measuring device. The counting device is used to count the number of revolutions of the anticoagulant pump and the blood pump within a specified time, and the volume measuring device is used to measure the volume of the anticoagulant passing through the anticoagulant pump and the volume of the blood passing through the blood pump within a specified time. To measure the volume of the anticoagulant passing through the anticoagulant pump, an air detector is respectively arranged in the anticoagulant pump tube and the blood pump tube, and the counting device counts the number of revolutions of the anticoagulant pump in the interval between non-detections of air by the air detectors in the anticoagulant pump tube and the blood pump tube. The volume of the anticoagulant passing through the anticoagulant pump is calculated according to the anticoagulant tube size as well as the tube length between both air detectors. To measure the volume of the blood passing through the blood pump, the counting device counts the number of revolutions of the blood pump in the interval between a non-detection of air in the blood tube by the air detector in the blood pump tube and reach of the set point of plasma mass. The sum of the blood volume in a centrifugal cup and the blood volume in the blood tube between the air detector and the plasma bag is the volume of the blood passing through the blood pump. The blood volume in the blood tube between the air detector and the plasma bag depends on the blood tube size and the tube length between the air detector and the plasma bag.

The flow rate measurement means of the utility model further comprises a counting device and a mass measurement device. The counting device is used for counting the number of revolutions of the anticoagulant pump and the blood pump within a specified time, and the mass measurement device is used to measure the anticoagulant dosage used in the anticoagulant bag within a specified time and the mass of plasma obtained by centrifuging blood in a centrifuge within the specified time.

An alarm device is arranged in the flow rate feedback assembly of the utility model and correspondingly connected with the determining module. The flow rate feedback assembly is capable of correcting the flow rate of the anticoagulant pump and the flow rate of the blood pump within ±20% of the set flow rate. When the flow rate of the anticoagulant pump or the blood pump exceeds the correctable range, the pump-controlled tube is considered to be abnormal, and the alarm device alerts the equipment user to check for tube blockage, pump roller failure or other abnormalities without delay. In this way, the reliability and maintainability of the plasmapheresis equipment are improved.

The flow rate feedback assembly of the utility model is jacketed on the anticoagulant tube and the blood tube in a simple way without introduction of contaminant into the collected blood, and the flow rate feedback assembly is directly compatible with the existing consumables.

The various aspects, embodiments, implementations or features of the utility model can be used separately or in any combination.

The utility model has a plurality of advantages. Different aspects, embodiments or implementations yield one or more of the following advantages. The utility model has an advantage that the flow rate of the anticoagulant pump and the flow rate of the blood pump are made steady near the set point by means of the flow rate feedback assembly. In this way, the precision control of the anticoagulant dosage for plasmapheresis is realized to improve the quality of plasmapheresis and reduce the impact on the health of the plasma donor. The utility model has another advantage that the flow rate feedback assembly of modular design is easy to be integrated with the plasmapheresis equipment of the prior art at a low cost and is applicable for a plurality of purposes.

The embodiments mentioned above are only preferred embodiments of the utility model and not used to limit the utility model. Any modification, equivalent replacement and improvement made within the spirit and rule of the utility model shall be incorporated in the protection scope of the utility model.

## Claims

1. A high-precision peristaltic pump flow rate control system, **characterized in that** the high-precision peristaltic pump flow rate control system comprises a motor, a peristaltic pump, a rotational speed feedback assembly, a flow rate feedback assembly and a controller; the motor is correspondingly connected with the peristaltic pump, the peristaltic pump is correspondingly connected with the rotational speed feedback assembly and the flow rate feedback assembly, the rotational speed feedback assembly and the flow rate feedback assembly are correspondingly connected with the controller, and the controller is correspondingly connected with the motor; and the flow rate feedback assembly comprises a flow rate measurement means and a determining module.

2. The high-precision peristaltic pump flow rate control system according to claim 1, **characterized in that** the flow rate measurement means is a flow transducer.

3. The high-precision peristaltic pump flow rate control system according to claim 1, **characterized in that** an alarm device is arranged in the flow rate feedback assembly.

4. The high-precision peristaltic pump flow rate control system according to claim 1, **characterized in that** the flow rate feedback assembly is jacketed on an anticoagulant tube and a blood tube.

5. The high-precision peristaltic pump flow rate control system according to claim 2, **characterized in that** a self-calibration module is arranged in the flow transducer.

6. The high-precision peristaltic pump flow rate control system according to claim 2, **characterized in that** the flow transducer is a small-diameter ultrasonic flow transducer with an inner diameter range of 3 to 5 mm and an outer diameter of 6 to 7 mm.
